# EUROPEAN PATENT SPECIFICATION

(11) **EP 1 575 628 B1**
(45) Date of publication and mention of the grant of the patent: **14.06.2006**
(21) Application number: 03752348.7
(22) Date of filing: 12.09.2003
(51) Int. Cl.: A61L 15/46, A61F 13/15, B32B 27/14

(54) **ABSORBENT ARTICLES THAT INCLUDE A STRETCHABLE SUBSTRATE HAVING ODOR CONTROL PROPERTIES**
ABSORBIERENDE GEGENSTÄNDE ENTHALDEND EINEN DEHNFÄHIGEN TRÄGERMATERIAL MIT GERUCHSKONTROLLEEIGENSCHAFTEN
ARTICLES ABSORBANTS COMPRENANT UN SUBSTRAT ETIRABLE QUI PRESENTE DES PROPRIETES D'ELIMINATION DES ODEURS

(30) Priority: 23.12.2002 US 328750
(43) Date of publication of application: 21.09.2005
(73) Proprietor: KIMBERLY-CLARK WORLDWIDE, INC., Neenah, WI 54956 (US)
(72) Inventor: LONG, Andrew, M., Appleton, WI 54915 (US); EDENS, Ronald, L., Cumming, GA 30041 (US); QUINCY, III, Roger, B., Cumming, GA 30040 (US); GADSBY, Elizabeth, D., Marietta, GA 30068 (US)
(74) Representative: Schuster, Thomas
(86) International application number: PCT/US2003/028823
(87) International publication number: WO 2004/060420

(56) References cited:
- EP-A- 1 157 672
- WO-A-00/45763
- WO-A-02/34512
- US-B1- 6 203 810
- US-B1- 6 475 600

## Description

### Background of the Invention

Odor control agents have been conventionally incorporated into materials for a variety of reasons. For instance, activated carbon has been used to reduce a broad spectrum of odors in absorbent materials. However, one significant problem associated with many materials that contain odor control agents, particularly those that have a layered structure, is that the materials tend to act as a "barrier" to the efficacy of the odor control agent in reducing odor by preventing the malodors from fully interacting with the odor control agent. Even for substrates on which an odor control agent is disposed so that it is increasingly exposed to the malodorous environment, its odor control properties often become depleted before use. In addition, some odor control agents also cause a substantial increase in the stiffness of the material, thereby reducing its ability to function in a variety of applications.

As such, a need currently exists for an improved technique for incorporating an odor control agent into a material.

### Summary of the Invention

In accordance with one embodiment of the present invention, an absorbent article is disclosed that contains a substrate (e.g., film, fibrous material, foam, and so forth) that is, upon application of a force, stretchable to a stretched, biased length that is at least about 125% of its unstretched length. In some embodiments, the absorbent article may comprise an absorbent core disposed between a liquid permeable top sheet and a back sheet, the substrate forming at least a portion of the top sheet, the absorbent core, the back sheet, or combinations thereof. For instance, the absorbent article may be a personal care absorbent article selected from the group consisting of diapers, training pants, absorbent underpants, adult incontinence products, sanitary napkins, and so forth.

Regardless of the construction of absorbent article, the stretchable substrate is applied with an odor control material that is increasingly exposable to malodors when the substrate is stretched. For example, the odor control material may be selected from the group consisting of odor adsorbents, masking agents, odor inhibitors, odor neutralizers, and combinations thereof. Examples of suitable odor adsorbents include, for instance, activated carbon, zeolites, silica, clays, alumina, magnesia, titania, cyclodextrin and derivatives thereof, and combinations thereof. In some embodiments, the odor control material comprises from about 0.05% to about 20%, and in some embodiments, from about 1 % to about 5% by weight of the substrate.

A wide variety of materials and methods may be used to form the stretchable substrate. For example, in one embodiment, the stretchable substrate includes an elastic polymer. If desired, the elastic polymer may be contained within an elastic laminate, such as a neck-bonded laminate, stretch-bonded laminate, necked stretched-bonded laminate, and combinations thereof.

To apply the odor control material to the substrate, a variety of techniques may be utilized. For instance, in one embodiment, the odor control material is at least partially coated with a thermoplastic polymer. In such instances, the thermoplastic polymer coating may be bonded to a polymer of the substrate. Moreover, in one embodiment, the substrate contains at least one liquid-impermeable material, wherein the odor control material is applied to the liquid-impermeable material. If desired, the liquid-impermeable material may be breathable.

Other features and aspects of the present invention are discussed in greater detail below.

### Brief Description of the Drawings

A full and enabling disclosure of the present invention, including the best mode thereof, directed to one of ordinary skill in the art, is set forth more particularly in the remainder of the specification, which makes reference to the appended figures in which:
Fig. 1 illustrates a perspective view of an absorbent article that may be formed according to one embodiment of the present invention; and
Fig. 2 illustrates a perspective view of an absorbent article that may be formed according to another embodiment of the present invention.

### Detailed Description of Representative Embodiments

### Definitions

As used herein, the term "absorbent article" refers to any article capable of absorbing water or other fluids. Examples of some absorbent articles include, but are not limited to, personal care absorbent articles, such as diapers, training pants, absorbent underpants, fenestration materials, adult incontinence products, feminine hygiene products (e.g., sanitary napkins), and so forth; wound coverings; wipers; bed pads; shoe pads; clothing articles, such as perspiration pads, disposable swimming apparel, and so forth; air and water filtration devices; and so forth. Materials and processes suitable for forming such absorbent articles are well known to those skilled in the art.

As used herein the term "nonwoven fabric or web" means a web having a structure of individual fibers or threads which are interlaid, but not in an identifiable manner as in a knitted fabric. Nonwoven fabrics or webs have been formed from many processes such as for example, meltblowing processes, spunbonding processes, bonded carded web processes, etc.

As used herein, the term "meltblown fibers" refers to fibers formed by extruding a molten thermoplastic material through a plurality of fine, usually circular, die capillaries as molten fibers into converging high velocity gas (e.g. air) streams that attenuate the fibers of molten thermoplastic material to reduce their diameter, which may be to microfiber diameter. Thereafter, the meltblown fibers are carried by the high velocity gas stream and are deposited on a collecting surface to form a web of randomly disbursed meltblown fibers. Such a process is disclosed, for example, in U.S. Pat. No. 3,849,241 to Butin, et al.

Generally speaking, meltblown fibers may be microfibers that may be continuous or discontinuous, are generally smaller than 10 microns in diameter, and are generally tacky when deposited onto a collecting surface.

As used herein, the term "spunbonded fibers" refers to small diameter substantially continuous fibers that are formed by extruding a molten thermoplastic material from a plurality of fine, usually circular, capillaries of a spinnerette with the diameter of the extruded fibers then being rapidly reduced as by, for example, eductive drawing and/or other well-known spunbonding mechanisms. The production of spun-bonded nonwoven webs is described and illustrated, for example, in U.S. Patent Nos. 4,340,563 to Appel, et al., 3,692,638 to Dorschner, et al., 3,802,817 to Matsuki, et al., 3,338,992 to Kinney, 3,341,394 to Kinney, 3,502,763 to Hartman, 3,502,538 to Levy, 3,542,615 to Dobo, et al., and 5,382,400 to Pike, et al., Spunbond fibers are generally not tacky when they are deposited onto a collecting surface. Spunbond fibers can sometimes have diameters less than about 40 microns, and are often between about 5 to about 20 microns.

As used herein, the term "coform" generally refers to composite materials comprising a mixture or stabilized matrix of thermoplastic fibers and a second non-thermoplastic material. As an example, coform materials may be made by a process in which at least one meltblown die head is arranged near a chute through which other materials are added to the web while it is forming. Such other materials may include, but are not limited to, fibrous organic materials such as woody or non-woody pulp such as cotton, rayon, recycled paper, pulp fluff and also superabporbent particles, inorganic absorbent materials, treated polymeric staple fibers and so forth. Some examples of such coform materials are disclosed in U.S. Patent Nos. 4,100,324 to Anderson, et al.; 5,284,703 to Everhart, et al.; and 5,350,624 to Georger, et al.

### Detailed Description

Reference now will be made in detail to various embodiments of the invention, one or more examples of which are set forth below. Each example is provided by way of explanation of the invention, not limitation of the invention. Features illustrated or described as part of one embodiment, can be used on another embodiment to yield a still further embodiment. Thus, it is intended that the present invention covers such modifications and variations as come within the scope of the appended claims and their equivalents.

In general, the present invention is directed to a stretchable substrate applied with an odor control material. As a result of the present invention, it has been discovered that a substrate can be formed that is flexible and also capable of providing full exposure of the odor control material to malodors. Generally speaking, any of a variety of odor control materials may be utilized in the present invention. The odor control material may be provided in any form suitable for use in a substrate including, but not limited to, particles, fibers, flakes, filaments, spheres, and so forth. Although not required, particulate odor control materials generally have particle sizes ranging from about 1 to about 1000 microns. Liquid and/or semi-soiid odor controi materials, as well as coatings formed by drying liquid and/or semi-solid odor control materials, may also be used in the present invention.

For example, in one embodiment, odor adsorbents may be used. Some examples of suitable odor adsorbents include, but are not limited to, activated carbon, zeolites, silica, clays (e.g., smectite clay), alumina, magnesia, titania, cyclodextrin and derivatives thereof, combinations thereof, and so forth. For instance, suitable forms of activated carbon and techniques for formation thereof are described in U.S. Patent No. 5,834,114 to Economy, et al.; WO 01/97972 to Economy, et al.; and U.S. Patent Publication No. 2001/0024716. Some commercially available examples of activated carbon are made from saw dust, wood, charcoal, peat, lignite, bituminous coal, coconut shells, and so forth. One particular example of activated carbon that may be used in the present invention is Nuchar® RGC 40, a granular activated carbon available from MeadWestvaco Corp. RGC 40 can be obtained with a U.S. Mesh Size of 40 x 100 (150 to 425 microns), and can be ground to any desired median particle size, such as about 1 micron.

Further, odor-adsorbing forms of zeolites are also well known in the art. For instance, zeolites generally have an aluminate/silicate framework, with associated cations, M, providing overall electrical neutrality. Empirically, the zeolite framework can be represented as follows:

xAlO₂ . ySiO₂

with the electrically neutral zeolite represented as follows:

x/n M. xAlO₂. ySiO₂. zH₂O

wherein, x and y are each integers, M is a cation, and n is the charge on the cation. As noted by the empirical formula, zeolites may also contain water (zH₂O). M can be a wide variety of cations, e.g., Na⁺, K⁺, NH₄⁺, alkylammonium, heavy metals, and so forth. Still other forms of suitable zeolites may be described in U.S. Patent No. 6,096,299 to Guarracino, et al.

Moreover, some examples of cyclodextrins that may be suitable for use in the present invention include, but are not limited to, α-cyclodextrin, hydroxyalkyl α-cyclodextrin, alkyl α-cyclodextrin, β-cyclodextrin, hydroxyalkyl β-cyclodextrin, alkyl β-cyclodextrin, γ-cyclodextrin, hydroxyalkyl Y-cyclodextrin, and alkyl Y-cyclodextrin.

Furthermore, various other materials can also be applied to inhibit or prevent odor. For instance, certain antioxidant compounds can be used to inhibit the formation of odiferous compounds on the human body. For example, apocrine sweat is an initially odorless sweat containing proteins, pyrodextrose, iron ions, ammonia, and lipofuscin compounds. Moreover, sebaceous sweat glands produce sebum, which contains triglycerides, wax esters, squalene, and cholesterol. Various bacteria located on the surface of human skin will typically transform some of these components into unsaturated, longer chain fatty acid compounds, which are later oxidized into odiferous, saturated, shorter chain fatty acid compounds. As such, antioxidants can generally be utilized to prevent the oxidation of unsaturated, longer chain fatty acid compounds into saturated, shorter chain fatty acids, thereby inhibiting odor production. Some examples of antioxidants that can be utilized in a softening composition of the present invention include, but are not limited- to, Vitamin E, Vitamin E derivatives, Vitamin C, Vitamin C derivatives, Vitamin A palmitate, butylated hydroxy toluene, phenols, phenolic derivatives, thiodipropionate esters, hydroquinone derivatives, alkylated aryl amine, combinations thereof, and so forth.

In addition, some odor inhibitors can kill or prevent the growth of bacteria or other microbes that produce malodors and/or inhibit their growth. Some examples of such odor-inhibiting antimicrobial compounds indude, but are not limited to, bisphenols, such as Triclosan; quaternary ammonium compounds, such as benzalkonium chloride; esters of parahydroxy benzoic acid, such as methyl parabens; formaldehyde and formaldehyde donors, such as 2-bromo-2-nitro-1,3 propanediol, diazolidinyl urea, and imidazolidinyl urea; alkylisothizaolinones; phenoxyethanol; silver ions; natural or herbal antimicrobials (e.g. green tea) halogenated compounds; halomines; combinations thereof, and so torth. Other examples of suitable odor inhibitors can include various metallic compounds that are capable of inhibiting the activity of certain bacterial exo-enzymes that can result in the production of malodor. For example, exo-enzymes, such as aryl-sulfatases and beta-glucuronidases, are required to break down the steroids secreted with apocrine sweat and sebum, which results in malodor. Thus, by inhibiting the activity of such exo-enzymes, malodor can be inhibited. For instance, in one embodiment, metallic compounds, such as zinc and zinc compounds (e.g., zinc glycinate) can be utilized in the present invention to inhibit microbial activity on the skin. Other suitable odor inhibitors may include chelating or complexihg agents, such as ethylenediamines, ethylenediaminetetraacetic acids (EDTA) acid and/or salts thereof, citric acids and/or salts thereof, glucuronic acids and/or salts thereof, polyphosphates, organo phosphates, dimercaprols, and so forth.

Odor neutralizers may also be utilized. For instance, some suitable odor neutralizers may include, but are not limited to, sodium bicarbonate, polyacrylic acid, citric acid, boric acid, and so forth. In addition, odor control materials that "mask" odor, i.e.,masking agents, can also be used in the present invention. Some examples of suitable masking agents can include, but are not limited to, benzyl acetate, benzyl salicylate, iso-bomyl acetate, p-t-butyl cyclohexyl acetate, cetronellol, hexyl cinnamic aldehyde, indole, phenyl ethyl alcohol, vanillin, combinations thereof, and so forth. Moreover, in some instances, certain masking agents can also act as odor inhibitors. Examples of odor inhibitors that may also be able to mask odors include, but are not limited to, essential oils containing eugenol or phenol functionalities, such as clove oil, pimento oil, citronellol, geraniol, rose oil, cassia oil, sandlewood oil, undecyl alcohol, undecylenic alcohol, eugenol, combinations thereof, and so forth. Masking can be achieved through adding a fragrance or through use of a malodor-counteracting compound, such as those available from Flavor and Fragrance Specialties of Mahwah NJ.

In general, the amount of the odor control material utilized can vary depending on the nature of the materials selected and the desired level of odor control. In most embodiments, the odor control material is applied in an amount of from about 0.05% by weight to about 20% by weight, in some embodiments from about 0.5% by weight to about 10% by weight, and in some embodiments, from about 1% by weight to about 5% by weight of the substrate. In fact, it is believed that even very small amounts of an odor control material can significantly improve the odor properties of the substrate. Further, the odor control material may be applied, uniformly or non-uniformly, to the surface of the substrate and/or embedded therein.

vanous substrates may be incorporated with an odor control material in accordance with the present invention. For instance, nonwoven fabrics, woven fabrics, knit fabrics, wet-strength paper, films, foams, etc., may be applied with the odor control material. When utilized, the nonwoven fabrics may include, but are not limited to, spunbonded webs (apertured or non-apertured), meltblown webs, bonded carded webs, air-laid webs, coform webs, hydraulically entangled webs, and so forth. Regardless of the material selected, the substrate is "stretchable", i.e., upon application of a force, the substrate may be stretched to a biased length that is at least about 125%, and in some embodiments, at least about 150% its unstretched length. Optionally, the stretchable substrate is "elastic" in that it will also recover at least about 50% of its elongation upon release of the stretching, biasing force. In some instances, an elastic material can enhance the stretchability of the substrate by enabling it to be more easily bent and distorted. When present in a substrate, the elastic material takes on various forms. For example, the elastic material can make up the entire substrate or form, a portion of the substrate. In some embodiments, for instance, the elastic material can contain elastic strands or sections uniformly or randomly distributed throughout the substrate. Alternatively, the elastic material can be an elastic film or an elastic nonwoven web. The elastic material can also be a single layer or a multi-layered material.

In general; any material known in the art to possess elastic characteristics can be used in the present invention. For example, suitable elastic resins include block copolymers having the general formula A-B-A' or A-B, where A and A' are each a thermoplastic polymer endblock which contains a styrenic moiety and where B is an elastic polymer midbtock such as a conjugated diene or a lower alkene polymer. Block copolymers for the A and A' blocks, and the present block copolymers are intended to embrace linear, branched and radial block copolymers. In this regard, the radial block copolymers may be designated (A-B)m-X, wherein X is a polyfunctional atom or molecule and in which each (A-B)m- radiates from X in a way that A is an endblock. In the radial block copolymer, X may be an organic or inorganic polyfunctional atom or molecule and m may be an integer having the same value as the functional group originally present in X, which is usually at least 3, and is frequently 4 or 5, but not limited thereto. Thus, the expression "block copolymer," and particularly "A-B-A" and "A-B" block copolymers, can include all block copolymers having such rubbery blocks and thermoplastic blocks as discussed above, which can be extruded (e.g., by meltblowing), and without limitation as to the number of blocks. For example, elastic materials, such as (polystyrene/poly(ethytene-butylene)/ polystyrene) block copolymers, can be utilized. Commercial examples of such elastic copolymers are, for example, those known as KRATON® materials which are available from Kraton Polymers of Houston, Texas. KRATON® block copolymers are available in several different formulations, a number of which are identified in U.S. Patent Nos. 4,663,220, 4,323,534, 4,834,738, 5,093,422 and 5,304,599

Polymers composed of an elastic A-B-A-B tetrablock copolymer may also be used. Such polymers are discussed in U.S. Patent No. 5,332,613 to Taylor et al. In these polymers, A is a thermoplastic polymer block and B is an isoprene monomer unit hydrogenated to substantially a poly(ethylene-propylene) monomer unit. An example of such a tetrabtock copotymer is a styrene-poly(ethylenepropylene)-styrene-poly(ethylene-propylene) or S-EP-S-EP elastic block copolymer available from the Kraton Polymers of Houston, Texas under the trade designation KRATON® G-1657.

Other exemplary elastic materials that may be used include polyurethane elastic materials such as, for example, those available under the trademark ESTANE® from B.F. Goodrich & Co. or MORTHANE® from Morton Thiokol Corp., and polyester elastic materials such as, for example, copolyesters available under the trade designation HYTREL® from E.I. DuPont De Nemours & Company and copolyesters known as ARNITEL®, formerly available from Akzo Plastics of Amhem, Holland and now available from DSM of Sittard, Holland.

In addition, some examples of suitable elastic olefin polymers are available from Exxon Chemical Company of Baytown, Texas under the trade name ACHIEVE® for polypropylene based polymers and EXACT® and EXCEED® for polyethylene based polymers. Dow Chemical Company of Midland, Michigan has polymers commercially available under the name ENGAGE®. These materials are believed to be produced using non-stereoselective metallocene catalysts. Exxon generally refers to their metallocene catalyst technology as "single site" catalysts, while Dow refers to theirs as "constrained geometry" catalysts under the name INSIGHT® to distinguish them from traditional Ziegler-Natta catalysts that have multiple reaction sites. Further, other suitable elastic materials may include highly saturated nitrile polymers; silicone polymers; polyacrylate elastomers; and other elastomers and flexible polymers known in the art. Still other suitable elastic materials that may be used in the present invention include diblock, triblock, or multi-block elastic copolymers, such as olefinic copolymers (e.g., styrene-isoprene-styrene, styrene-butadiene-styrene, styrene-ethylene/butylene-styrene, or styrene-ethylene/propylene-styrene); polyurethanes; polyamides; polyesters; and so forth. Other examples of suitable elastic materials are described in U.S. Patent No. 6,362,389 to McDowall, et al.

When incorporating an elastic material, such as described above, into a substrate, it is sometimes desired that the elastic material be contained within an elastic laminate that also contains one or more other layers, such as foams, films, apertured films, and/or nonwoven webs. An elastic laminate generally contains layers that can be bonded together so that at least one of the layers has the characteristics of an elastic polymer. The elastic material used in the elastic laminates can be made from materials, such as described above, that are formed into films, such as a microporous film; fibrous webs, such as a web made from meltblown or spunbond fibers; foams; and so forth.

For example, in one embodiment, the elastic laminate can be a "neck-bonded" laminate. A "neck-bonded" laminate refers to a composite material having at least two layers in which one layer is a necked, non-elastic layer and the other layer is an elastic layer. The resulting laminate is thereby a material that is elastic in the cross-direction and/or machine-direction. Some examples of neck-bonded laminates are described in U.S. Patent Nos. 5,226,992, 4,981,747, 4,965,122, and 5,336,545, all to Morman The elastic laminate can also be a "stretch-bonded" laminate, which refers to a composite material having at least two layers in which one layer is a gatherable layer and in which the other layer is an elastic layer. The layers are joined together when the elastic layer is in an extended condition so that upon relaxing the layers, the gatherable layer is gathered. For example, one elastic member can be bonded to another member while the elastic member is extended at least about 25 percent of its relaxed length. Such a multilayer composite elastic material may be stretched until the nonelastic layer is fully extended.

For example, one suitable type of stretch-bonded laminate is a spunbonded laminate, such as disclosed in U.S. Patent No. 4,720,415 to VanderWielen et al. Another suitable type of stretch-bonded laminate is a continuous filament spunbonded laminate, such as disclosed in U.S. Patent No. 5,385,775 to Wright. For instance, Wright discloses a composite elastic material that includes: (1) an anisotropic elastic fibrous web having at least one layer of elastic meltblown fibers and at least one layer of elastic filaments autogenously bonded to at least a portion of the elastic meltblown fibers, and (2) at least one gatherable layer joined at spaced-apart locations to the anisotropic elastic fibrous web so that the gatherable layer is gathered between the spaced-apart locations. The gatherable layer is joined to the elastic fibrous web when the elastic web is in a stretched condition so that when the elastic web relaxes, the gatherable layer gathers between the spaced-apart bonding locations. Other composite elastic materials are described and disclosed in U.S. Patent Nos. 4,789,699 to Kieffer et al., 4,781,966 to Taylor, 4,657,802 to Morman, and 4,655,760 to Morman et al.

In one embodiment, the elastic laminate can also be a necked stretch bonded laminate. As used herein, a necked stretch bonded laminate is defined as a laminate made from the combination of a neck-bonded laminate and a stretch-bonded laminate. Examples of necked stretch bonded laminates are disclosed in U.S. Patent Nos. 5,114,781 and 5,116,662. Of particular advantage, a necked stretch bonded laminate can be stretchable in both the machine and cross-machine directions.

The substrate can further include a moisture barrier. In one embodiment, the moisture barrier can be made from liquid-impermeable plastic films, such as polyethylene and polypropylene films. Generally, such plastic films are impermeable to gases and water vapor, as well as liquids, in addition, in other embodiments, it may be desired that one or more of the layers of the substrate be impermeable to liquids, but permeable to gases and water vapor (i.e., breathable). For example, the substrate may contain a breathable material, such as films, nonwoven webs, nonwovens coated with a film-forming material (e.g., an emulsion coated onto a meltblown web or a laminate comprising spunbond and meltblown webs), combinations thereof, and so forth. For example, a film may be constructed with micropores therein to provide breathability. The micropores form what is often referred to as tortuous pathways through the film. Liquid contacting one side of the film does not have a direct passage through the film. Instead, a network of microporous channels in the film prevents liquids from passing, but allows gases and water vapor to pass. Another type of film that may be used is a nonporous, continuous film, which, because of its molecular structure, is capable of forming a vapor-pervious barrier. Among the various polymeric films that fall into this type include films made from a sufficient amount of poly(vinyl alcohol), polyvinyl acetate, ethylene vinyl alcohol, polyurethane, ethylene methyl acrylate, and ethylene methyl acrylic acid to make them breathable. Without intending to be held to a particular mechanism of operation, it is believed that films made from such polymers solubilize water molecules and allow transportation of those moiecules from one surface of the film to the other. Accordingly, these films may be sufficiently continuous, i.e., nonporous, to make them liquid-impermeable, but still allow for vapor permeability.

In some embodiments, the breathable material may be made from polymer films that contain a filler, such as calcium carbonate. As used herein, a "filler" generally refers to particulates and other forms of materials that may be added to the film polymer extrusion blend and that will not chemically interfere with the extruded film, but which may be uniformly dispersed throughout the film, Generally the fillers are in particulate form an have a spherical or non-spherical shape with average particle sizes in the range of from about 0.1 to about 7 microns. Both organic and inorganic fillers are contemplated to be within the scope of the present invention. Examples of suitable fillers include; but are not limited to, calcium carbonate, various kinds of clay, silica, alumina, barium carbonate, sodium carbonate, magnesium carbonate, talc, barium sulfate, magnesium sulfate, aluminum sulfate, titanium dioxide, zeolites, cellulose-type powders, kaolin, mica, carbon, calcium oxide, magnesium oxide, aluminum hydroxide, pulp powder, wood powder, cellulose derivatives, chitin and chitin derivatives. A suitable coating, such as, for example, stearic acid, may also be applied to the filler particles if desired.

These films are made breathable by stretching the filled films to create the microporous passageways as the polymer breaks away from the calcium carbonate during stretching. For example, in one embodiment, the breathable material contains a stretch-thinned film that includes at least two basic components, i.e., a polyolefin polymer and a filler. These components are mixed together, heated, and then extruded into a film layer using any one of a variety of film-producing processes known to those of ordinary skill in the film processing art. Such film-making processes include, for example, cast embossed, chill and flat cast, and blown film processes.

Generally, on a dry weight basis, based on the total weight of the film, the stretch-thinned film includes from about 30% to about 90% by weight of a polyolefin polymer. In some embodiments, the stretch-thinned film includes from about 30% to about 90% by weight of a filler. Examples of such films are described in U.S. Patent Nos. 5,843,057 to McCormack; 5,855,999 to McCormack; 6,002,064 to Kobylivker, et al.; and 6,037,281 to Mathis, et al. In one embodiment, the polyolefin polymer may be a predominately linear polyolefin polymer, such as linear, tow-density polyethylene (LLDPE) or polypropylene. The term "linear low density polyethylene" refers to polymers of ethylene and higher alpha olefin comonomers, such as C₃- C₁₂ and combinations thereof, having a Melt Index (as measured by ASTM D-1238) of from about 0.5 to about 10 grams per 10 minutes at 190°C. Moreover, the term "predominately linear" means that the main polymer chain is linear with less than about 5 long chain branches per 1000 ethylene units. Long chain branches include, for example, carbon chains greater than C₁₂. For predominately linear polyolefin polymers that are nonelastic, short chain branching (C₃ -C₁₂) due to comonomer inclusion is typically less than about 20 short chains per 1000 ethylene units and about 20 or greater for polymers that are elastic. Examples of predominately linear polyolefin polymers include, without limitation, polymers produced from the following monomers: ethylene, propylene, 1-butene, 4-methyl-pentene, 1-hexene, 1-octene and higher olefins as well as copolymers and terpolymers of the foregoing. In addition, copolymers of ethylene and other olefins including butene, 4-methyl-pentene, hexene, heptene, octene, decene, etc., are also examples of predominately linear polyolefin polymers.

As mentioned herein, the stretch-thinned film may be formed using any conventional process known to those familiar with film formation. The polyolefin polymer and filler are mixed in the desired proportions and then heated and extruded into a film. To provide more uniform breathability, as reflected by the water vapor transmission rate of the film, the filler may be uniformly dispersed throughout the polymer blend and, consequently, throughout the film layer itself. The film may be uniaxially or biaxially stretched. The film may be uniaxially stretched, for example, to about 1.1 to about 7.0 times its original length. In some embodiments, the film may be stretched to about 1.5 to about 6.0 times its original length. Further, in some embodiments, the film may be stretched to about 2.5 to about 5.0 times its original length. The film may alternatively be biaxially stretched using techniques familiar to one of ordinary skill in the art.

Breathable films, such as described above, may constitute the entire breathable material, or may be part of a multilayer film. Multilayer films may be prepared by cast or blown film coextrusion of the layers, by extrusion coating, or by any conventional layering process. Further, other breathable materials that may be suitable for use in the present invention are described in U.S. Patent Nos. 4,341,216 to Obenour, 4,758,239 to Yeo, et al.; 5,628,737 to Dobrin, et al.; 5,836,932 to Buell; 6,114,024 to Forte; 6,153,209 to Vega, et al.; 6,198,018 to Curro; 6,203,810 to Alemany, et at; and 6,245,401 to Ying, et al.

If desired, the breathable film may also be bonded to a nonwoven fabric, knitted fabric, and/or woven fabric using well-known techniques. For instance, suitable techniques for bonding a film to a nonwoven web are described in U.S. Patent Nos. 5,843,057 to McCormack; 5,855,999 to McCormack; 6,002,064 to Kobylivker, et al.; 6,037,281 to Mathis, et al.; and WO 99/12734. In one embodiment, for example, a breathable, liquid-impermeable, breathable film/nonwoven laminate material may be formed from a nonwoven layer and a breathable film layer. The layers may be arranged so that the breathable film layer is attached to the nonwoven layer.

In addition to the films mentioned above, various other breathable films can be utilized in the present invention. One type of film that may be used is a nonporous, continuous film, which, because of its molecular structure, is capable of forming a vapor-permeable barrier. Among the various polymeric films that fall into this type include films made from a sufficient amount of poly(vinyt alcohol), polyvinyl acetate, ethylene vinyl alcohol, polyurethane, ethylene methyl acrylate, and ethylene methyl acrylic acid to make them breathable. Although the inventors do not intend to be held to a particular mechanism of operation, it is believed that films made from such polymers solubilize water molecules and allow transportation of those molecules from one surface of the film to the other. Accordingly, such films may be sufficiently continuous, i.e., nonporous, to make them liquid-impermeable but still allow for vapor permeability.

Regardless of the particular substrate selected, it is typically desired that the odor control material be applied thereto in such a manner that it becomes increasingly exposed to the environment where the malodors exist only upon stretching of the substrate. For example, in one embodiment, the odor control material is partially or completely applied with a thermoplastic polymeric coating that is permeable or impermeable to liquids and/or moisture. For instance, some thermoplastic polymers suitable for such a coating can include, but are not limited to, polyolefins (e.g., polyethylene, polypropylene, polybutylene, etc.), including homopolymers, copolymers, terpolymers and blends thereof; ethylene vinyl acetate; ethylene ethyl acrylates; ethylene acrylic acids; ethylene methyl acrylates; ethylene normal butyt acryfates; polyurethanes; poly(vinyl) chtorides; polyesters; polyamides; polystyrenes; poly(vinyl) alcohols; copolymers, terpolymers, and blends of the foregoing; and so forth. The thermoplastic polymer coating may be softened or melted so that it bonds to a polymer of the stretchable substrate. Thus, in this manner, the odor control material becomes adhesively attached to the surface of the substrate. In addition, upon stretching of the substrate, the polymer coating can tear, thereby allowing the odor control material to be increasingly exposed to the environment where the malodors exist. Specifically, it is believed that the bonds formed between the crossover points of the thermoplastic polymer coating and the polymer of the stretchable material will be stronger than the tear strength of the thermoplastic polymer coating. Thus, the coating will rupture before the coated odor control material becomes detached.

Any bonding method may generally be utilized in the present invention to bond together the thermoplastic polymer coating and the polymer of the stretchable substrate. For instance, thermal bonding techniques, such as thermal point bonding, pattern unbonding, etc., through-air bonding, and ultrasonic bonding are some examples of techniques that may be utilized, In addition, other bonding methods, such as adhesive bonding, etc., may also be utilized. For example, some suitable adhesives are described in U.S. Patent Nos. 5,425,725 to Tanzer, et al.; 5,433,715 to Tanzer, et. al.; and 5,593,399 to Tanzer, et al. In one embodiment, for instance, a separate thermoplastic polymer may act as an adhesive between the coated odor control material and the polymer of the stretchable substrate.

In another embodiment, the odor control material may be contained or embedded with the polymer matrix of the stretchable material. Thus, upon stretching, the odor control material becomes increasingly exposed to the environment where the malodors are present. For example, in one embodiment, the odor control material is incorporated into an elastic laminate that contains a liquid-impermeable layer bonded to an elastic layer. The liquid-impermeable layer may be, for instance, a vapor-impermeable film or a breathable film. In one embodiment, the odor control material may be mixed with the polymer used to form the liquid-impermeable layer so that it is contained therein upon formation. Once bonded to an elastic layer, the resulting fibrous laminate is stretchable. The substrate may be configured so that the elastic layer faces the area in which malodors are generally produced. Thus, upon stretching, the elastic layer extends and its structure opens, thereby allowing the malodors to be placed in communication with the odor control material contained with the liquid-impermeable layer bonded to the elastic layer.

In general, the stretchable substrate, such as described above, may be utilized in a wide variety of applications in which odor control is benefcial. For example, the stretchable substrate may be incorporated into an absorbent article, such as personal care absorbent articles (e.g., diapers, training pants, incontinence devices) and feminine hygiene products (e.g., sanitary napkins), where it can function to reduce odor. For example, the stretchable substrate may be incorporated into regions of the absorbent article that are likely to remain relatively dry during use, such as the wings of a sanitary napkin, waistbands or leg cuffs of a diaper, or the remote ends or sides of an article positioned a certain distance from the zone configured to receive bodily fluids. By being positioned in a relatively dry area of the absorbent article, the stretchable substrate can reduce the odor of the article without having its odor-reducing capabilities substantially weakened by wetting. The stretchable substrate may form the entire absorbent article, or may form only a portion of the article.

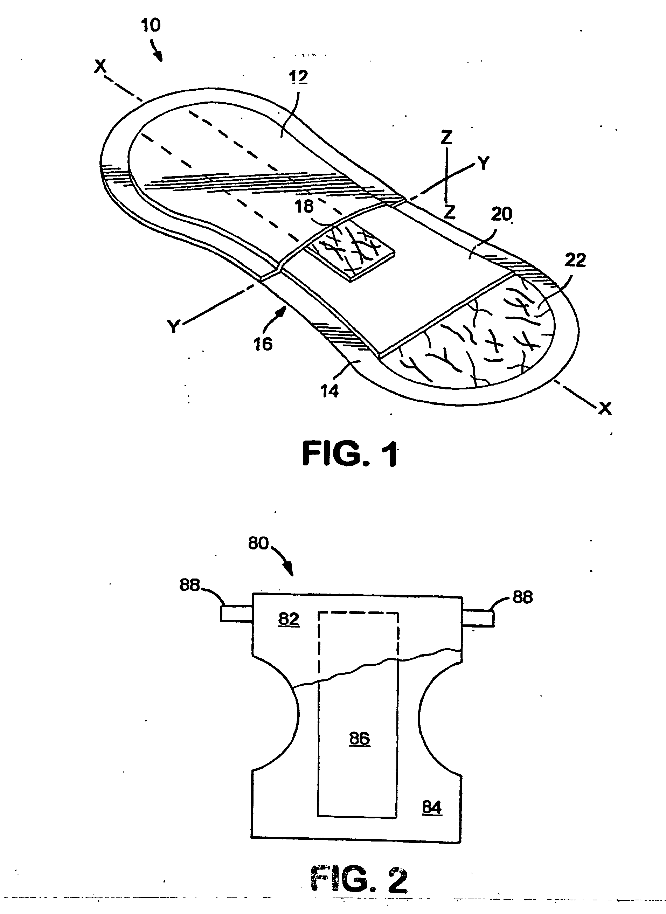

Referring to Fig. 1, for example, one embodiment of such a sanitary napkin includes a top sheet 12, a back sheet 14, and an absorbent core 16, any of which may contain the stretchable substrate of the present invention. The absorbent core 16 is positioned inward from the outer periphery of the sanitary napkin 10 and includes a body-facing surface positioned adjacent the top sheet 12 and a garment-facing surface positioned adjacent the back sheet 14. The top sheet 12 is generally designed to contact the body of the user and is liquid-permeable. The top sheet 12 can surround the absorbent core 16 so that it completely encases the absorbent article 10. Alternatively, the top sheet 12 and the back sheet 14 can extend beyond the absorbent core 16 and be peripherally joined together, either entirely or partially, using known techniques. Typically, the top sheet 12 and the back sheet 14 are joined by adhesive bonding, ultrasonic bonding, or any other suitable joining method known in the art. The top sheet 12 can also contain a plurality of apertures (not shown) formed therethrough to permit body fluid to pass more readily into the absorbent core 16. The apertures can be randomly or uniformly arranged throughout the top sheet 12, or they can be located only in the narrow longitudinal band or strip arranged along the longitudinal axis X-X of the absorbent article 10. The apertures permit rapid perletration of body fluid down into the absorbent core 16. The size, shape, and diameter of any number of apertures can be varied to suit one's particular needs.

The back sheet 14 is generally liquid-impermeable and designed to face the inner surface, i.e., the crotch portion of an undergarment (not shown). The back sheet 14 can permit a passage of air or vapor out of the absorbent article 10, while still blocking the passage of liquids.

In the illustrated embodiment, the absorbent core 16 contains three separate and distinct absorbent members 18,20 and 22, any of which may contain the stretchable substrate of the present invention. It should be understood, however, that any number of absorbent members can be utilized in the present invention. For example, in one embodiment, only the absorbent member 22 may be utilized. As shown, the first absorbent member 18, or intake member, is positioned between the top sheet 12 and the second absorbent member 20, or transfer delay member. The intake member 18 represents a significant absorbing portion of the absorbent article 10 and has the capability of absorbing at least about 80%, particularly about 90%, and more particularly about 95% of the body fluid deposited onto the absorbent artide 10. In terms of amount of body fluid, the intake member 18 can absorb at least about 20 grams, particularly about 25 grams, and more particularly, about 30 or more grams of body fluid.

The intake member 18 can generally have any shape and/or size desired. For example, in one embodiment, the intake member 18 has a rectangular shape, with a length equal to or less than the overall length of the absorbent artide 10, and a width less than the width of the absorbent article 10. For example, a length of between about 150 mm to about 300 mm and a width of between about 10 mm to about 40 mm can be utilized.

Typically, the intake member 18 is made of a material that is capable of rapidly transferring, in the z-direction, body fluid that is delivered to the top sheet 12. Because the intake member 18 is generally of a dimension narrower than the absorbent article 10, the sides of the intake member 18 are spaced away from the longitudinal sides of the absorbent article 10 and the body fluid is restricted to the area within the periphery of the intake member 18 before it passes down and is absorbed into the transfer delay member 20. This design enables the body fluid to be combined in the central area of the absorbent article 10 and to be wicked downward.

A second absorbent member 20, or transfer delay member, is also, positioned vertically below the intake member 18. In some embodiments, the transfer delay member 20 contains a material that is less hydrophilic than the other absorbent members, and may generally be characterized as being substantially hydrophobic. The transfer delay member 20 can generally have any size, such as a length of about 150 mm to about 300 mm. Typically, the length of the transfer delay member 20 is approximately equal to the length of the absorbent article 10. The transfer delay member 20 can also be equal in width to the intake member 18, but is typically wider. For example, the width of the transfer delay member 20 can be from between about 50 mm to about 75 mm, and particularly about 48 mm.

Besides the above-mentioned members, the absorbent core 16 also includes a composite member 22. For example, the composite member 22 can be a coform material. In this instance, fluids can be wicked from the transfer delay member 20 into the absorbent member 22. The composite absorbent member 22 may be formed separately from the intake member 18 and/or transfer delay member 20, or can be formed simultaneously therewith.

The absorbent article 10 may also contain other components as well. For instance, in some embodiments, the lower surface of the back sheet 14 can contain an adhesive for securing the absorbent article 10 to an undergarment. In such instances, a backing (not shown) may be utilized to protect the adhesive side of the absorbent article 10 so that the adhesive remains clean prior to attachment to undergarment. The backing can generally have any desired shape or dimension. For instance, the backing can have a rectangular shape with dimension about 17 to about 21 cm in length and about 6.5 to 10.5 cm in width. The backing is designed to serve as a releasable peel strip to be removed by the user prior to attachment of the absorbent article 10 to the undergarment. The backing serving as a releasable peel strip can be a white Kraft paper that is coated on one side so that it can be released readily from the adhesive side of the absorbent article 10. The coating can be a silicone coating, such as a silicone polymer commercially available from Akrosil of Menasha, Wisconsin.

Once formed, the absorbent article 10 generally functions to absorb and retain fluids, such as menses, blood, urine, and other excrements discharged by the body during a menstrual period. For example, the intake member 18 can allow the body fluid to be wicked downward in the z-direction and away from the top sheet 12 so that the top sheet 12 retains a dry and comfortable feel to the user. Moreover, the intake member 18 can also absorb a significant amount of the fluid. The transfer delay member 20 initially accepts fluid from the intake member 18 and then wicks the fluid along its length and width (-x and -y axis) before releasing the fluid to the composite absorbent member 22. The composite absorbent member 22 then wicks the fluid along its length and width (-x and -y axis) utilizing a greater extent of the absorbent capacity than the transfer delay member 20. Therefore, the composite absorbent member 22 can become completely saturated before the fluid is taken up by the transfer delay member 20. The fluid is also wicked along the length of the transfer delay member 20 and the composite absorbent member 22, thereby keeping the fluid away from the edges of the absorbent article 10. This allows for a greater utilization of the absorbent core 16 and helps reduce the likelihood of side leakage.

As indicated above, other types of adsorbent articles may also be formed in the present invention. Referring to Fig. 2, for example, one embodiment of a diaper 80 is illustrated that includes a liquid permeable top sheet or liner 82, a back sheet or outercover 84, and an absorbent core 86 disposed between and contained by the top sheet 82 and back sheet 84. The diaper 80 may also include a fastening device 88, such as adhesive fastening tapes or mechanical hook and loop type fasteners. The stretchable substrate may be used to form various portions of the diaper 80 including, but not limited to, the top sheet 82, the back sheet 84, and the absorbent core 86.

For instance, other absorbent configurations are described in U.S. Patent Nos. 5,197,959 to Buell; 5,085,654 to Buell; 5,634,916 to Lavon, et al.; 5,569,234 to Buell, et al.; 5,716,349 to Taylor, et al.; 4,950,264 to Osborn, III; 5,009,653 to Osborn, III; 5,509,914 to Osborn, III; 5,649,916 to DiPalma, et al.; 5,267,992 to Van Tillburg; 4,687,478 to Van Tillburg; 4,285,343 to McNair; 4,608,047 to Mattingly; 5,342,342 to Kitaoka; 5,190,563 to Herron, et al.; 5,702,378 -to Widlund, et al.; 5,308,346 to Sneller, et al.; 6,1.10,158 to Kielpikowski; and WO 99/00093 to Patterson, et al. For instance, in one embodiment, the stretchable substrate of the present invention For instance, in one embodiment, the stretchable substrate of the present invention is used to form the leg cuff of a diaper.

Thus, as a result of the present invention, a substrate may be formed that is capable of increasingly exposing an odor control material contained therein to malodors. In particular, upon stretching the substrate, the odor control material is increasingly exposed to the environment in which the malodors exist. Further, because the odor control material typically remains largely unexposed prior to stretching, its odor control properties are not generally expended through exposure during storage and shipment prior to sale and use, which can result in longer stability and better performance of the odor control material. In addition, the stiffness often associated with substrates that contain odor control agents, such as activated carbon, can be substantially inhibited in the present invention.

## Claims

1. An absorbent article that contains a substrate that is, upon application of a force, stretchable to a stretched, biased length that is at least 125% of its unstretched length, wherein said stretchable substrate is applied with an odor control material that is increasingly exposable to malodors when said substrate is stretched.

2. An absorbent article as defined in claim 1, wherein said odor control material is selected from the group consisting of odor adsorbents, masking agents, odor inhibitors, odor neutralizers, and combinations thereof.

3. An absorbent article as defined in claim 1, wherein said odor control material is an odor inhibitor selected from the group consisting of antioxidants, antimicrobial compounds, chelating agents, and combinations thereof.

4. An absorbent article as defined in claim 1, wherein said odor control material is an odor adsorbent

5. An absorbent article as defined in claim 4, wherein said odor adsorbent is selected from the group consisting of activated carbon, zeolites, silica, clays, alumina, magnesia, titania, cyclodextrin and derivatives thereof, and combinations thereof.

6. An absorbent article as defined in claim 1, wherein said odor control material comprises from 0.05% to 20% by weight of said substrate.

7. An absorbent article as defined in claim 1, wherein said odor control material comprises from 1% to 5% by weight of said substrate.

8. An absorbent article as defined in claim 1, wherein said stretchable substrate includes an elastic polymer.

9. An absorbent article as defined in claim 8, wherein said substrate is an elastic laminate.

10. An absorbent article as defined in claim 9, wherein said elastic laminate is selected from the group consisting of neck-bonded laminates, stretch-bonded laminates, necked stretched-bonded laminates, and combinations thereof.

11. An absorbent article as defined in claim 1, wherein said odor control material is at least partially coated with a thermoplastic polymer.

12. An absorbent article as defined in claim 11, wherein said thermoplastic polymer is bonded to a polymer of said substrate.

13. An absorbent article as defined in claim 1, wherein said substrate contains at least one liquid-impermeable material.

14. An absorbent article as defined in claim 13, wherein said liquid-impermeable material is breathable.

15. An absorbent artide as defined in claim 13, wherein said odor control material is applied to said liquid-impermeable material.

16. An absorbent article as defined in claim 1, wherein the absorbent article comprises an absorbent core disposed between a liquid permeable top sheet and a back sheet, said substrate forming at least a portion of said top sheet, said absorbent core, said back sheet, or combinations thereof.

17. An absorbent article as defined in claim 1, wherein the absorbent article is a personal care absorbent article selected from the group consisting of diapers, training pants, absorbent underpants, adult incontinence products, sanitary napkins, and combinations thereof.

18. A personal care absorbent article that comprises an absorbent core disposed between a liquid permeable top sheet and a back sheet, wherein an elastic substrate forms at least a portion of said top sheet, said absorbent core, said back sheet, or combinations thereof, said elastic substrate being, upon application of a force, stretchable to a stretched, biased length that is at least 125% of its unstretched length and capable of recovering at least 50% of its elongation upon release of said force, said elastic substrate being applied with from 0.05% to 20% of an odor control material that is increasingly exposable to malodors when said substrate is stretched.

19. A personal care absorbent article as defined in claim 18, wherein said odor control material is selected from the group consisting of odor adsorbents, masking agents, odor inhibitors, odor neutralizers, and combinations thereof.

20. A personal care absorbent article as defined in claim 18, wherein said odor control material is an odor adsorbent selected from the group consisting of activated carbon, zeolites, silica, clays, alumina, magnesia, titania, cyclodextrin and derivatives thereof, and combinations thereof.

21. A personal care absorbent article as defined in claim 18, wherein said odor control material comprises from 1% to 5% by weight of said elastic substrate.

22. A personal care absorbent article as defined in claim 18, wherein said elastic substrate includes an elastic polymer.

23. A personal cars absorbent article as defined in claim 22, wherein said odor control material is at least partially coated with a thermoplastic polymer that is bonded to said elastic polymer of said elastic substrate.

24. A personal care absorbent article as defined in claim 18, wherein said elastic substrate is an elastic laminate.

25. A personal care absorbent article as defined in claim 24, wherein said elastic laminate is selected from the group consisting of neck-bonded laminates, stretch-bonded laminates, necked stretched-bonded laminates, and combinations thereof.

26. A personal care absorbent article as defined in claim 18, wherein said elastic substrate contains at least one liquid-impermeable material.

27. A personal care absorbent article as defined in claim 26, wherein said odor control material is applied to said liquid-impermeable material.

28. A personal care absorbent article as defined in claim 18, wherein the personal care absorbent article is selected from the group consisting of diapers, training pants, absorbent underpants, adult incontinence products, sanitary napkins, and combinations thereof.

29. An elastic substrate that is, upon application of a force, stretchable to a stretched, biased length that is at least 125% of its unstretched length and capable of recovering at least 50% of its elongation upon release of said force, said elastic substrate being applied with from 0.05% to 20% of an odor control material that is exposable to malodors when said substrate is stretched.

30. An elastic substrate as defined in claim 29, wherein said odor control material is selected from the group consisting of odor adsorbents, masking agents, odor inhibitors, odor neutralizers, and combinations thereof.

31. An elastic substrate as defined in claim 29, wherein said odor control material is an odor adsorbent selected from the group consisting of activated carbon, zeolites, silica, clays, alumina, magnesia, titania, cyclodextrin and derivatives thereof, and combinations thereof.

32. An elastic substrate as defined in claim 29, wherein said odor control material comprises from 1% to 5% by weight of said elastic substrate.

33. An elastic substrate as defined in claim 29, wherein said elastic substrate includes an elastic polymer.

34. An elastic substrate as defined in claim 33, wherein said odor control material is at least partially coated with a thermoplastic polymer that is bonded to said elastic polymer of the elastic substrate.

35. An elastic substrate as defined in claim 29,wherein said elastic substrate is an elastic laminate.

36. An elastic substrate as defined in claim 35, wherein said elastic laminate is selected from the group consisting of neck-bonded laminates, stretch-bonded laminates, necked stretched-bonded laminates, and combinations thereof.

37. An elastic substrate as defined in claim 29, wherein said elastic substrate contains at least one fiquid-impemzeable material.

38. An elastic substrate as defined in claim 37, wherein said odor control material is applied to said liquid-impermeable material.

39. An elastic substrate as defined in claim 38, wherein said liquid-impermeable material is breathable.

## Patentansprüche

1. Ein absorbierender Artikel der ein Substrat enthält, das bei Einwirkung einer Kraft bis zu einer gedehnten, gespannten Länge, die mindestens 125% seiner ungedehnten Länge beträgt, dehnbar ist, wobei das dehnbare Substrat mit einem Geruchskontrollmaterial versehen ist, das Gerüchen zunehmend aussetzbar ist, wenn das Substrat gedehnt ist.

2. Ein absorbierender Artikel wie in Anspruch 1 definiert, wobei das Geruchskontrollmaterial ausgewählt ist aus der Gruppe bestehend aus Geruchsabsorbierern, Überdeckungsmitteln, Geruchshemmem, Geruchsneutralisierern, und Kombinationen aus diesen.

3. Ein absorbierender Artikel wie in Anspruch 1 definiert, wobei das Geruchskontrollmaterial ein Geruchshemmer ist, ausgewählt aus der Gruppe bestehend aus Antioxidanten, antimikrobiellen Verbindungen, chelatbildenden Verbindungen, und Kombinationen aus diesen.

4. Ein absorbierender Artikel wie in Anspruch 1 definiert, wobei das Geruchskontrollmaterial ein Geruchsabsorbierer ist.

5. Ein absorbierender Artikel wie in Anspruch 4 definiert, wobei der Geruchsabsorbierer ausgewählt ist aus der Gruppe bestehend aus Aktivkohle, Zeoliten, Silika, Ton, Tonerde, Magnesiumoxid, Titandioxid, Cyclodextrin und seinen Derivaten, und Kombinationen aus diesen.

6. Ein absorbierender Artikel wie in Anspruch 1 definiert, wobei das Geruchskontrollmaterial von 0,05% bis 20% Gewichtsanteil des Substrats umfasst.

7. Ein absorbierender Artikel wie in Anspruch 1 definiert, wobei das Geruchskontrollmaterial von 1% bis 5% Gewichtsanteil des Substrats umfasst.

8. Ein absorbierender Artikel wie in Anspruch 1 definiert, wobei das dehnbare Substrat ein elastisches Polymer beinhaltet.

9. Ein absorbierender Artikel wie in Anspruch 8 definiert, wobei das Substrat ein elastisches Laminat ist.

10. Ein absorbierender Artikel wie in Anspruch 9 definiert, wobei das elastische Laminat ausgewählt ist aus der Gruppe bestehend aus eingeschnürt gebundenen (neck-bonded) Laminaten, gedehnt gebundenen (stretch-bonded) Laminaten, eingeschnürt gedehnt gebundenen (necked stretch-bonded) Laminaten, und Kombinationen aus diesen.

11. Ein absorbierender Artikel wie in Anspruch 1 definiert, wobei das Geruchskontrollmaterial mindestens teilweise mit einem thermoplastischen Polymer beschichtet ist.

12. Ein absorbierender Artikel wie in Anspruch 11 definiert, wobei das thermoplastische Polymer an ein Polymer des Substrats gebunden ist.

13. Ein absorbierender Artikel wie in Anspruch 1 definiert, wobei das Substrat mindestens ein flüssigkeitsundurchlässiges Material enthält.

14. Ein absorbierender Artikel wie in Anspruch 13 definiert, wobei das flüssigkeitsundurchlässige Material atmungsaktiv ist.

15. Ein absorbierender Artikel wie in Anspruch 13 definiert, wobei das flüssigkeitsundurchlässige Material mit dem Geruchskontröllmaterial versehen ist.

16. Ein absorbierender Artikel wie in Anspruch 1 definiert, wobei der absorbierende Artikel ein absorbierendes Innenteil zwischen einem flüssigkeitsdurchlässigen oberen Lage und einer unteren Lage umfasst, und wobei das Substrat mindestens einen Teil der oberen Lage, des absorbierenden Innenteils, der unteren Lage oder Kombinationen aus diesen bildet.

17. Ein absorbierender Artikel wie in Anspruch 1 definiert, wobei der absorbierende Artikel ein absorbierender Körperpflege-Artikel ist, ausgewählt aus der Gruppe bestehend aus Windeln, Trainingshosen, absorbierenden Unterhosen, Inkontinenzprodukten für Erwachsene, Damenbinden, und Kombinationen aus diesen.

18. Ein absorbierender Körperpflege-Artikel, welcher ein absorbierendes Innenteil umfasst, das zwischen einer flüssigkeitsdurchlässigen oberen Lage und einer unteren Lage bereitgestellt ist, wobei ein elastisches Substrat mindestens einen Teil der oberen Lage, des absorbierenden Innenteils, der unteren Lage oder Kombinationen aus diesen bildet, und wobei das elastische Substrat bei Einwirkung einer Kraft, dehnbar ist bis zu einer dehnbaren, gespannten Länge, die mindestens 125% seiner ungedehnten Länge beträgt, und geeignet ist, bei Entlastung der Kraft mindestens 50% seiner Dehnung wieder einzuholen, und wobei das elastische Substrat zu 0,05% bis zu 20% mit einem Geruchskontrollmaterial versehen ist, das Gerüchen zunehmend aussetzbar ist, wenn das Substrat gedehnt ist.

19. Ein absorbierender Körperpflege-Artikel wie in Anspruch 18 definiert, wobei das Geruchskontrollmaterial ausgewählt ist aus der Gruppe bestehend aus Geruchsabsorbierem, Überdeckungsmitteln, Geruchshemmem, Geruchsneutralisierern, und Kombinationen aus diesen.

20. Ein absorbierender Körperpflege-Artikel wie in Anspruch 18 definiert, wobei das Geruchskontrollmaterial ein Geruchsabsorbierer ist, ausgewählt aus der Gruppe bestehend aus Aktivkohle, Zeoliten, Silika, Ton, Tonerde, Magnesiumoxid, Titandioxid, Cyclodextrin und seinen Derivaten, und Kombinationen aus diesen.

21. Ein absorbierender Körperpflege-Artikel wie in Anspruch 18 definiert, wobei das Geruchskontrollmaterial von 1 % bis 5% Gewichtsanteil des elastischen Substrats umfasst.

22. Ein absorbierender Körperpflege-Artikel wie in Anspruch 18 definiert, wobei das elastische Substrat ein elastisches Polymer beinhaltet.

23. Ein absorbierender Körperpflege-Artikel wie in Anspruch 22, wobei das Geruchskontrollmaterial mindestens teilweise mit einem thermoplastischen Polymer beschichtet ist, das an das elastische Polymer des elastischen Substrates gebunden ist.

24. Ein absorbierender Körperpflege-Artikel wie in Anspruch 18 definiert, wobei das elastische Substrat ein elastisches Laminat ist.

25. Ein absorbierender Körperpflege-Artikel wie in Anspruch 24 definiert, wobei das elastische Laminat ausgewählt ist aus der Gruppe bestehend aus eingeschnürt gebundenen (neck-bonded) Laminaten, gedehnt gebundenen (stretch-bonded) Laminaten, eingeschnürt gedehnt gebundenen (necked stretch-bonded) Laminaten, und Kombinationen aus diesen.

26. Ein absorbierender Körperpflege-Artikel wie in Anspruch 18 definiert, wobei das elastische Substrat mindestens ein flüssigkeitsundurchlässiges Material enthält.

27. Ein absorbierender Körperpflege-Artikel wie in Anspruch 26 definiert, wobei das flüssigkeitsundurchlässige Material mit dem Geruchskontrollmaterial versehen ist.

28. Ein absorbierender Körperpflege-Artikel wie in Anspruch 18 definiert, wobei der absorbierende Körperpflege-Artikel ausgewählt ist aus der Gruppe bestehend aus Windeln, Trainingshosen, absorbierenden Unterhosen, Inkontinenzprodukten für Erwachsene, Damenbinden, und Kombinationen aus diesen.

29. Ein elastisches Substrat, welches bei Einwirkung einer Kraft dehnbar ist bis zu einer gedehnten, gespannten Länge, die mindestens 125% seiner ungedehnten Länge beträgt, und welches geeignet ist, bei Entlastung der Kraft mindestens 50% seiner Dehnung wieder einzuholen, und wobei das elastische Substrat zu 0,05% bis zu 20% mit einem Geruchskontrollmaterial versehen ist, das Gerüchen aussetzbar ist, wenn das Substrat gedehnt ist.

30. Ein elastischen Substrat wie in Anspruch 29 definiert, wobei das Geruchskontrollmaterial ausgewählt ist aus der Gruppe bestehend aus Geruchsabsorbierem, Überdeckungsmitteln, Geruchshemmem, Geruchsneutralisierern, und Kombinationen aus diesen.

31. Ein elastisches Substrat wie in Anspruch 29 definiert, wobei das Geruchskontrollmaterial ein Geruchsabsorbierer ist, ausgewählt aus der Gruppe bestehend aus Aktivkohle, Zeoliten, Silika, Ton, Tonerde, Magnesiumoxid, Titandioxid, Cyclodextrin und seinen Derivaten, und Kombinationen aus diesen.

32. Ein elastisches Substrat wie in Anspruch 29 definiert, wobei das Geruchskontrollmaterial von 1 % bis 5% Gewichtsanteil des elastischen Substrats umfasst.

33. Ein elastisches Substrat wie in Anspruch 29 definiert, wobei das elastische Substrat ein elastisches Polymer beinhaltet.

34. Ein elastisches Substrat wie in Anspruch 33 definiert, wobei das Geruchskontrollmaterial mindestens teilweise mit einem thermoplastischen Polymer beschichtet ist, das an das elastische Polymer des elastischen Substrates gebunden ist.

35. Ein elastisches Substrat wie in Anspruch 29 definiert, wobei das elastische Substrat ein elastisches Laminat ist.

36. Ein elastisches Substrat wie in Anspruch 35 definiert, wobei das elastische Laminat ausgewählt ist aus der Gruppe bestehend aus eingeschnürt gebundenen (neck-bonded) Laminaten, gedehnt gebundenen (stretch-bonded) Laminaten, eingeschnürt gedehnt gebundenen (necked stretch-bonded) Laminaten, und Kombinationen aus diesen.

37. Ein elastisches Substrat wie in Anspruch 29 definiert, wobei das elastische Substrat mindestens ein flüssigkeitsundurchlässiges Material enthält.

38. Ein elastisches Substrat wie in Anspruch 37 definiert, wobei das flüssigkeitsundurchlässige Material mit dem Geruchskontrollmaterial versehen ist.

39. Ein elastisches Substrat wie in Anspruch 38 definiert, wobei das flüssigkeitsundurchlässige Material atmungsaktiv ist.

## Revendications

1. Article absorbant qui contient un substrat qui, lors de l'application d'une force, est extensible jusqu'à une longueur étirée, sollicitée, égale à au moins 125 % de sa longueur non-étirée, article dans lequel ledit substrat extensible a reçu une application d'un matériau de maîtrise des odeurs qui est de plus en plus exposable à de mauvaises odeurs lorsque ledit substrat est étiré.

2. Article absorbant tel que défini dans la revendication 1, dans lequel ledit matériau de maîtrise des odeurs est sélectionné dans le groupe consistant en les adsorbeurs d'odeurs, les agents masquants, les inhibiteurs d'odeurs, les neutraliseurs d'odeurs, et leurs combinaisons.

3. Article absorbant tel que défini dans la revendication 1, dans lequel ledit matériau de maîtrise des odeurs est un inhibiteur d'odeurs sélectionné dans le groupe consistant en les antioxydants, les composés antimicrobiens, les agents chélatants et leurs combinaisons.

4. Article absorbant tel que défini dans la revendication 1, dans lequel ledit matériau de maîtrise des odeurs est un adsorbeur d'odeurs.

5. Article absorbant tel que défini dans la revendication 4, dans lequel ledit adsorbeur d'odeurs est sélectionné dans le groupe consistant en le carbone activé, les zéolites, la silice, les argiles, l'alumine, la magnésie, l'oxyde de titane, la cyclodextrine et ses dérivés, et les combinaisons de ceux-ci.

6. Article absorbant tel que défini dans la revendication 1, dans lequel ledit matériau de maîtrise des odeurs constitue de 0,05 % à 20 % en poids dudit substrat.

7. Article absorbant tel que défini dans la revendication 1, dans lequel ledit matériau de maîtrise des odeurs constitue de 1 % à 5 % en poids dudit substrat.

8. Article absorbant tel que défini dans la revendication 1, dans lequel ledit substrat extensible inclut un polymère élastique.

9. Article absorbant tel que défini dans la revendication 8, dans lequel ledit substrat est un laminé élastique.

10. Article absorbant tel que défini dans la revendication 9, dans lequel ledit laminé élastique est sélectionné dans le groupe consistant en les laminés liés sous striction, les laminés liés sous extension, les laminés liés sous extension/liés sous striction, et leurs combinaisons.

11. Article absorbant tel que défini dans la revendication 1, dans lequel ledit matériau de maîtrise des odeurs est au moins partiellement revêtu d'un polymère thermoplastique.

12. Article absorbant tel que défini dans la revendication 11, dans lequel ledit polymère thermoplastique est lié à un polymère dudit substrat.

13. Article absorbant tel que défini dans la revendication 1, dans lequel ledit substrat contient au moins un matériau imperméable aux liquides.

14. Article absorbant tel que défini dans la revendication 13, dans lequel ledit matériau imperméable aux liquides est respirant.

15. Article absorbant tel que défini dans la revendication 13, dans lequel ledit matériau de maîtrise des odeurs est appliqué audit matériau imperméable aux liquides.

16. Article absorbant tel que défini dans la revendication 1, dans lequel ledit article absorbant comprend un noyau absorbant disposé entre une feuille supérieure perméable aux liquides et une feuille-dossier, ledit substrat formant au moins une portion de ladite feuille supérieure, dudit noyau absorbant, de ladite feuille-dossier, ou de combinaisons de ceux-ci.

17. Article absorbant tel que défini dans la revendication 1, dans lequel ledit article absorbant est un article absorbant d'hygiène intime sélectionné dans le groupe consistant en les changes pour nourrissons, les culottes d'apprentissage de la propreté, les sous-vêtements absorbants, les produits pour l'incontinence adulte, les serviettes hygiéniques, et leurs combinaisons.

18. Article absorbant d'hygiène intime qui comprend un noyau absorbant disposé entre une feuille supérieure perméable aux liquides et une feuille-dossier, dans lequel un substrat élastique forme au moins une portion de ladite feuille supérieure, dudit noyau absorbant, de ladite feuille-dossier, ou de combinaisons de ceux-ci, ledit substrat élastique étant, lors de l'application d'une force, extensible jusqu'à une longueur étirée, sollicitée, qui est égale à au moins 125 % de sa longueur non-étirée, et étant capable de récupérer au moins 50 % de son allongement lorsque cesse ladite force, ledit substrat élastique ayant reçu une application allant de 0,05 % à 20 % d'un matériau de maîtrise des odeurs qui est de plus en plus exposable à de mauvaises odeurs lorsque ledit substrat est étiré.

19. Article absorbant d'hygiène intime tel que défini dans la revendication 18, dans lequel ledit matériau de maîtrise des odeurs est sélectionné dans le groupe consistant en les adsorbeurs d'odeurs, les agents masquants, les inhibiteurs d'odeurs, les neutraliseurs d'odeurs, et leurs combinaisons.

20. Article absorbant d'hygiène intime tel que défini dans la revendication 18, dans lequel ledit matériau de maîtrise des odeurs est un adsorbeur d'odeurs sélectionné dans le groupe consistant en le carbone activé, les zéolites, la silice, les argiles, l'alumine, la magnésie, l'oxyde de titane, la cyclodextrine et ses dérivés, et les combinaisons de ceux-ci.

21. Article absorbant d'hygiène intime tel que défini dans la revendication 18, dans lequel ledit matériau de maîtrise des odeurs constitue de 1 % à 5 % en poids dudit substrat élastique.

22. Article absorbant d'hygiène intime tel que défini dans la revendication 18, dans lequel ledit substrat élastique inclut un polymère élastique.

23. Article absorbant d'hygiène intime tel que défini dans la revendication 22, dans lequel ledit matériau de maîtrise des odeurs est au moins partiellement revêtu par un polymère thermoplastique qui est lié audit polymère élastique dudit substrat élastique.

24. Article absorbant d'hygiène intime tel que défini dans la revendication 18, dans lequel ledit substrat élastique est un laminé élastique.

25. Article absorbant d'hygiène intime tel que défini dans la revendication 24, dans lequel ledit laminé élastique est sélectionné dans le groupe consistant en les laminés liés sous striction, les laminés liés sous extension, les laminés liés sous extension/liés sous striction, et leurs combinaisons.

26. Article absorbant d'hygiène intime tel que défini dans la revendication 18, dans lequel ledit substrat élastique contient au moins un matériau imperméable aux liquides.

27. Article absorbant d'hygiène intime tel que défini dans la revendication 26, dans lequel ledit matériau de maîtrise des odeurs est appliqué audit matériau imperméable aux liquides.

28. Article absorbant d'hygiène intime tel que défini dans la revendication 18, dans lequel ledit article absorbant d'hygiène intime est sélectionné dans le groupe consistant en les changes pour nourrissons, les culottes d'apprentissage de la propreté, les sous-vêtements absorbants, les produits pour l'incontinence adulte, les serviettes hygiéniques, et leurs combinaisons.

29. Substrat élastique qui, lors de l'application d'une force, est extensible jusqu'à une longueur étirée, sollicitée, égale à au moins 125 % de sa longueur non-étirée, et qui est capable de récupérer au moins 50 % de son allongement lorsque cesse ladite force, ledit substrat élastique ayant reçu une application allant de 0,05 % à 20 % d'un matériau de maîtrise des odeurs qui est exposable à de mauvaises odeurs lorsque ledit substrat est étiré.

30. Substrat élastique tel que défini dans la revendication 29, dans lequel ledit matériau de maîtrise des odeurs est sélectionné dans le groupe consistant en les adsorbeurs d'odeurs, les agents masquants, les inhibiteurs d'odeurs, les neutraliseurs d'odeurs, et leurs combinaisons.

31. Substrat élastique tel que défini dans la revendication 29, dans lequel ledit matériau de maîtrise des odeurs est un adsorbeur d'odeurs sélectionné dans le groupe consistant en le carbone activé, les zéolites, la silice, les argiles, l'alumine, la magnésie, l'oxyde de titane, la cyclodextrine et ses dérivés, et les combinaisons de ceux-ci.

32. Substrat élastique tel que défini dans la revendication 29, dans lequel ledit matériau de maîtrise des odeurs constitue de 1 % à 5 % en poids dudit substrat élastique.

33. Substrat élastique tel que défini dans la revendication 29, dans lequel ledit substrat élastique inclut un polymère élastique.

34. Substrat élastique tel que défini dans la revendication 33, dans lequel ledit matériau de maîtrise des odeurs est au moins partiellement revêtu par un polymère thermoplastique qui est lié audit polymère élastique dudit substrat élastique.

35. Substrat élastique tel que défini dans la revendication 29, dans lequel ledit substrat élastique est un laminé élastique.

36. Substrat élastique tel que défini dans la revendication 35, dans lequel ledit laminé élastique est sélectionné dans le groupe consistant en les laminés liés sous striction, les laminés liés sous extension, les laminés liés sous extension/liés sous striction, et leurs combinaisons.

37. Substrat élastique tel que défini dans la revendication 29, dans lequel ledit substrat élastique contient au moins un matériau imperméable aux liquides.

38. Substrat élastique tel que défini dans la revendication 37, dans lequel ledit matériau de maîtrise des odeurs est appliqué audit matériau imperméable aux liquides.

39. Substrat élastique tel que défini dans la revendication 38, dans lequel ledit matériau imperméable aux liquides est respirant.
